# EUROPEAN PATENT SPECIFICATION

(11) **EP 1 465 600 B1**
(45) Date of publication and mention of the grant of the patent: **07.03.2007**
(21) Application number: 02788202.6
(22) Date of filing: 19.12.2002
(51) Int. Cl.: A61K 9/14, A61K 9/20

(54) **PARTICULATE COMPOSITIONS COMPRISING A LIPOPHILIC LIQUID**
PARTIKULÄRE ZUSAMMENSETZUNG ENTHALTEND EINE LIPOPHILE FLÜSSIGKEIT
COMPOSITIONS PARTICULAIRES COMPRENANT UN LIQUIDE LIPOPHILE

(30) Priority: 20.12.2001 US 343142 P
(43) Date of publication of application: 13.10.2004
(73) Proprietor: Alpex Pharma SA, 6805 Mezzovico-vica (CH)
(72) Inventor: STROPPOLO, Federico, 6974 Aldesago (CH); CICCARELLO, Franco, CH-6809 Mezzovico (CH); MILANI, Rita, CH-6952 Canobbio (CH); BELLORINI, Lorenzo, I-21028 Travedona, Monate (IT)
(74) Representative: Minoja, Fabrizio
(86) International application number: PCT/GB2002/005824
(87) International publication number: WO 2003/053410

(56) References cited:
- WO-A-99/48469
- RO-B- 115 325
- US-A- 3 966 949
- ELSHABOURY M H ET AL: "Formulation and release kinetics of sustained release peppermint oil granules for treatment of the irritable Bowel syndrome" PHARMAZEUTISCHE INDUSTRIE 1992 GERMANY, vol. 54, no. 7, 1992, pages 645-647, XP008014789 ISSN: 0031-711X
- TAKASHIMA Y ET AL: "Reduction of tablet coloration at tableting for oily medicine (tocopheryl nicotinate)" INTERNATIONAL JOURNAL OF PHARMACEUTICS 1999 NETHERLANDS, vol. 187, no. 1, 1999, pages 125-135, XP002234126 ISSN: 0378-5173

## Description

### SUMMARY OF THE INVENTION

In accordance with this invention, there are provided solid particulates which are formed from a lipophilic liquid, a hydrophilic solid dispersant, and a solid particulating agent. Particulates disclosed herein are characterized by their liquid-retention properties, that is, the liquid constituent of the particulates does not exude from the particulates when they are subjected to pressure, for example, pressure which is sufficient to form a cohesive tablet from a powder form of the particulates.

There is provided also in accordance with the present invention, a tablet which has a friability of less than about one wt. % and which comprises a hydrophilic solid dispersant, a solid particulating agent, and a lipophilic liquid that is dispersed uniformly throughout said composition and is dispersible from said tablet upon contact with an aqueous media within about five minutes. In preferred form the lipophilic liquid is dispersible from the composition within about one minute after contacting the composition with an aqueous media.

Another aspect of the present invention is the provision of a method for forming a composition in solid form from a mixture of constituents comprising a lipophilic liquid, a hydrophilic solid dispersant, and a solid particulating agent. In preferred form, the composition is formed by adding the particulating agent to a mixture of the lipophilic liquid and the hydrophilic solid dispersant. Also, in preferred form, the composition of the present invention comprises a lipophilic liquid which is an oil that has bioactive properties, the particulating agent is silica and the hydrophilic solid dispersant is mannitol, wherein the weight ratio of lipophilic liquid and mannitol is 1:1, and the weight ratio of silica to the mixture of lipophilic agent and mannitol is 1:4.

### DETAILED DESCRIPTION OF THE INVENTION

There are a multitude of applications where it is desired to form a composition which is a solid at room temperature from a mixture of materials in which at least one of the materials is a liquid at room temperature. For a number of such applications, it is essential that the normally liquid constituent of the resulting solid be retained in the solid composition when it is subjected to pressure. As such, the solid composition should have liquid-retention properties.

In some applications, it is essential that the liquid material in the composition be dispersed from the solid composition within a relatively short time, for example, five minutes after contact with an aqueous medium, and in such applications, compositions which disperse the liquid material in one minute or less after contact with an aqueous media are preferred. As such, the solid composition should have also liquid-dispersing properties.

One example of the aforementioned types of applications is the provision of a tablet for oral ingestion in which the tablet comprises a normally liquid material which has bio-active properties, for example, pharmaceutical activity. Typically, a tablet is formed by compressing and shaping a powdery mixture of the materials comprising the tablet. The present invention can be utilized effectively to form tablets from a mixture of materials in which at least one of the materials is normally a liquid at room temperature.

It is believed that the present invention will be used widely in applications which involve the formation of tablets from a powdery mixture of materials which include a liquid material and a material which has bio-active properties. However, the present invention has broader applicability and, accordingly, involves the formation of a composition in the form of solid particulates which are formed from a mixture of materials comprising a lipophilic liquid, a hydrophilic solid dispersant, and a solid particulating agent. The term "particulates" refers to discrete particles, for example, pellets, beads, granules, and powders, the last mentioned preferably having an average particle size between about five and about 300 microns (µm).

The lipophilic liquid for use in the present invention comprises a material which is soluble in a non-polar solvent, for example, aliphatic organic solvents, fats, and oils, and is immiscible or partially immiscible in water, that is, its miscibility in water is not greater than about 0.01 wt. % e.g. at 25°C.

Lipophilic liquids suitable for use in the practice of the present development can be either a material which itself has desired properties or a material which functions as a carrier, for example, a solvent, for a material that has desired properties. The lipophilic liquid can be synthetic or naturally occurring or derived from natural sources. Silicone oils are an example of a class of synthetic materials which can function as a solvent or carrier of materials having desired properties. Examples of lipophilic liquids derived from natural sources that can function as a carrier or solvent are seed oils, for example, corn oil, and animal source oils, for example liver oil and squalene. Additional examples include oils from edible seeds and olive oil.

As mentioned above, it is believed that the present invention will be used widely in applications in which the solid composition has bioactive properties, that is, the composition includes one or more ingredients that impart to it bioactive properties. The bioactive material for use in the composition of the present invention is any substance which can be introduced into an animal's biological system via absorption from the gastrointestinal tract of the animal and which, upon absorption, elicits in the animal a therapeutic, prophylactic, diagnostic, or nutritional response.

WO99/48469 discloses tablets obtained from citric acid - sugar granules comprising a mixture of aerosil and spearmint oil.

Examples of bioactive properties are analgesic activity, anti-oxidant activity, vitamin supplementation, carminative, local anesthetic, expectorant, anthelmintic and local antiseptic.

For such bioactive applications, the lipophilic liquid comprises a bioactive material. The lipophilic liquid itself may have bioactive properties or bioactive material may be present as another liquid or as a solid that is dissolved in the lipophilic liquid. For example, there are naturally occurring oils which comprise a mixture of ingredients that include one or more solids that have bioactive properties and that are dissolved in the oil by an ingredient of the oil which is a solvent for the bioactive material. Bioactive lipophilic liquids include liquids which are obtained from natural sources, exemplified by essential oils as well as oils which can be synthesized, for example, vitamin A.

Essential oils are lipophilic liquids obtained from natural sources. They may be obtained by physical process such as, for example, pressing plant material or by extraction or distillation processes. In general, they are a mixture comprising one or more volatile oils and may also include other materials which are soluble in the volatile oil(s). Essential oils may comprise, as well, one or more constituents that have partial miscibility with water. Examples of essential oils include those which have organoleptic properties, for example, odor or taste properties as possessed, for example, by eucalyptus oil and those that have as well pharmaceutical properties, for example, as possessed by pine needle oil which has expectorant properties.

Waters, essences, and extracts exemplify essential oils which have some water miscibility, and in general comprise the essential oil, water, and a component which improves their solubility in water, for example, a glycol or an alcohol. They are obtained from plant materials using steam extraction/distillation (waters), alcohol extraction (essences), and extraction by a water/alcohol mixture (extracts). An example of a water is peppermint water. Examples of essences and extracts are valerian oil, eucalyptus oil, and fennel oil.

Other examples of essential oils include allspice, amber, american wormseed, angelica, anise, anise japanese, anthemis, arbor vitae, asarum, balm, basil, bay, bergamot, bitter almond, bitter orange, cade, cajeput, calamus, canphora, canada fleabane, canada snakeroot, caraway, cannabis, cardamon, cascarilla, cashew nut shell, cassia, castor, cedar leaf, cedar wood, celery, champaca, chenopodium, cherry laurel, chinese cinnamon, cinnamon, cinnamon ceylon, citronella, clove, copaiva, coriander, crisp mint, cubeb, cumin, curlet mint, cypress, dill, dwarf pine needles, egg yolk, erigeron, fennel, fir, fleabane, garlic, geranium, ginger, grapes, green oil, hedeoma, hops, hyssop, jojoba, juniper, lavender, lecithin, lemon, levant worm seed, linaloe, mace, majoran, melissa balm, mirbane, monarda, mountain pine, mustard expressed, myrcia, miristica, mirtle, meroli, niaoli, niove, nut, nutmeg, orange, orange flowers, origanum, palma christy, parsley, patchuli, pelargonium geranium, pennyroyal, pepper, peppermint, pettigrain, pimento, pine, pulegium, rice brain, rose, rose geranium, rosemary, rue, santal, sassafras, savin, scotch fir, silver fir, silver pine, spearmint, spike, sweet almonds, sweet bay, sweet flag, tansy, thuya, thyme, turpentine, valerian, vetiver, white cedar, wild marjoran, wine, wine heavy, worm wood, yarrow, sweet orange, white birch. A mixture of two or more lipophilic liquids can be used in formulating the composition of the present invention.

Mannitol, the hydrophilic dispersant for use in the practice of the present development, is a solid at room temperature (about 25 °C). The dispersant also has a water solubility at room temperature such that at minimum it meets the USP 24 definition of "freely soluble", that is, it requires no more than 10 weight parts of water to completely dissolve 1 weight part of dispersant, and the dispersant is sufficiently lipophilic to permit it to be "wetted" by the lipophilic liquid; however, its lipophilic properties are such that it is not soluble to any appreciable degree in the lipophilic liquid, that is, its solubility in the lipophilic liquid is no greater than about 0.1 wt. % at room temperature. It is believed that the lipophilic properties of the dispersant are associated with its functioning as a thickening agent for the lipophilic liquid. As example of the degree to which the lipophilic liquid and solid hydrophilic dispersant interact is that a mixture of the lipophilic liquid and the dispersant can have the characteristics of a flour dough of the type used in baked goods.

Without wanting to be bound by theory, it is believed that the solid hydrophilic dispersant functions in various ways in the formation of the composition of the present invention and its properties. It is believed that the dispersant helps the lipophilic liquid to "wet" the surface of the particulating agent (described below) during the formation of the solid particulates of the present invention. The ability of the dispersant to promote such "wetting" is believed to be important in promoting the homogeneous dispersion of the liquid constituent in the individual particulates comprising the composition. Additionally, it is believed that the dispersant functions to produce particulates that have a dry appearance and acceptable flowability (described below). This is in contrast to particulates that have poor flowability and an oily appearance. It is believed also that the dispersant plays a role in the ability of the lipophilic liquid to be retained in the composition, for example, when a powdery form thereof is subjected to pressure and shaped into a tablet. This is in contrast to particulates that exude oil when subjected to pressure.

Mannitol is believed also to aid in dispersing the lipophilic liquid from the solid form of the composition when it is contacted with an aqueous medium. As mentioned above, it is believed that the present invention will be used widely to form powders from which there are formed bioactive tablets for oral ingestion. In use, the tablets come into contact with aqueous-based body fluids. It is believed that the dispersant aids in dispersing the lipophilic liquid throughout the aqueous media when the tablet is contacted therewith. This is believed to be accomplished by virtue of the dispersant's interacting with the particulating agent whose surface has hydrophilic properties. It is thought that the surface of the particulating agent is wetted with the aqueous medium and that this aids in releasing the lipophilic liquid from the particulating agent.

If the hydrophilic dispersant does not promote cohesion, cohesion enhancers, for example, starch, can be added to formulations intended for use in forming tablets. This typically adds to the size of the tablet. Thus, a dispersant which also promotes cohesion is advantageous in reducing tablet size. This is a particular advantage when making tablets from particulates incorporating oils of low activity. The use of such oils requires that a relatively large amount of particulate be incorporated into the tablet, thus increasing size.

Silica for use as particulating agent suitable for use in the practice of the present invention may have various properties. One is a surface that can be wetted by both water and a solution of the hydrophilic solid dispersant. It can be characterized further in that, when placed in contact with an equal volume of neutral water, the pH of the water remains between about pH3 and about pH9. The particulating agent can be further characterized in that, upon contact with water, the surface of the agent undergoes a hydrolysis reaction. Additionally, a particulating agent for use in the practice of the present invention is compatible, i.e. does not react, with the lipophilic liquid constituent(s) of the composition.

Preferably, silica has a relatively high mass specific surface area, typically greater than about 25 m²/g. For applications in which the composition comprises an ingestible material, the particulating agent is preferably a material generally recognized as safe (GRAS) for ingestion.

An example of a suitable aerosil is Degussa Aerosil 200^{®} (Degussa).

The composition of the present invention can include other constituents which impart desired properties to the composition. Examples of such materials are flavoring agents, lubricants, sweetening agents, binders, fillers, taste masking-agents, and other excipients known in the art.

The composition of the present development is made by mixing the lipophilic liquid, the hydrophilic dispersing agent, and the particulating agent. In preferred form, the composition of the present invention can be prepared by admixing the particulating agent with a mixture comprising the lipophilic liquid and the hydrophilic liquid dispersant. The mixture of lipophilic liquid and dispersant can be prepared in any suitable way. For example, the dispersant can be added to the lipophilic liquid held, for example, in a mixing container or the lipophilic liquid can be added to the dispersant which is held in such container. The lipophilic liquid and dispersant are mixed for a sufficient period of time to produce a homogeneous composition, for example, a composition which is in the form of a batter-like, paste-like, or dough-like material comprising the constituents. Thereafter, the particulating agent is added to the lipophilic liquid/dispersant mixture (precursor composition) or the mixture is added to the particulating agent and the resulting composition is mixed until particulates comprising the aforementioned materials are obtained.

The preparation of particulate and precursor compositions can be effected at room temperature or ambient conditions and in conventional blending or mixing equipment. The particulate and precursor compositions can be prepared in equipment of any suitable scale, for example, in manual mixing equipment which relies on the use of a paddle or a spatula or in motorized equipment, for example, an electric mixer or blender. Inasmuch as the particulates can be prepared at room temperature, it is feasible to formulate the particulates from materials which are volatile or heat-sensitive.

The following guidelines are set forth for selecting the proportion of the ingredients that comprise the composition of the present invention. The lipophilic liquid should be present in an amount at least sufficient to impart to the composition those properties which are contributed by the lipophilic liquid to the composition. For use in bioactive compositions, the amount of the bioactive lipophilic liquid should be an amount at least sufficient for a dosage form thereof to exhibit the desired bioactive effect. The hydrophilic liquid dispersant should be used in an amount at least sufficient to convert the lipophilic liquid into a pourable thickened mass and the particulating agent should be present in an amount at least sufficient to convert the aforementioned thickened mass into a dry particulate mass having the liquid component homogeneously distributed therein.

These requirements are met by a composition wherein the lipophilic liquid and mannitol are combined in a weight ratio of 1:1 and wherein silica is combined with the mixture of lipophilic agent and mannitol in a weight ratio of silica to said mixture of 1:4.

In determining the maximum amount of each of the constituents comprising the composition, the following considerations should be taken into account. With respect to the lipophilic liquid, excessive amounts of the liquid will yield a material which exudes oil under pressure. Even greater excesses of the liquid will result in a particulate that is oily. With respect to the hydrophilic solid dispersant, an amount which provides a paste-like consistency should be used, with the maximum amount providing a mass which has shape-retaining properties, about the consistency of wet clay. An excessive amount of the dispersant, when mixed with the liquid component, will result in a mass which does not flow under pressure. With respect to the particulating agent, the maximum amount of agent usable is that amount that gives minimum average loading of the liquid component to the product particulate without resulting in a non-homogeneous distribution of the liquid components across the particulate mass.

It is believed that for most applications, the preferred composition will comprise about 25 wt. % to about 55 wt. % of the lipophilic liquid, about 25 wt. % to about 45 wt. % of the hydrophilic solid dispersant, and about 15 to about 30 wt. % of the particulating agent. For use in preparing particulates of the present invention that can be used in formulating tablets, a preferred formulation comprises the composition in which the weight ratio of hydrophilic dispersant to lipophilic liquid is between about 0.5 to 1 and about 1.5 to 1 and the weight ratio of lipophilic liquid to particulating agent is between 1 to 1 and about 3 to 1.

Generally speaking, the process of the present invention may yield particulates having a wide variation in average particle size, typically from about 5 microns to about 300 microns. It should be appreciated that such particulates will include particles having sizes outside of the aforementioned range, that is, some particles that are less than about 5 microns and some greater than about 300 microns. The particulates of the present invention can be separated into collections of particles having average particle sizes outside of the afore mentioned ranges, for example, by sieving or other means known in the art. In the case where it is desired to have a collection of particulates of relatively larger average particle size, the particulates of the present invention can be subjected to an agglomeration process, for example granulation, to produce particulates having an average particle size greater than 300 microns, while retaining the essential features described above. Thus, particulates can be agglomerated to form beads or pellets.

Powders for use in forming tablets in accordance with the present invention will typically have an average particle size of about 50 microns to about 200 microns, and may be obtained by the process described above, for example, by sieving the particulates to select the fraction having the desired average particle size.

The particulates of the present development, and thereby the liquid constituents contained there, can be formulated into compositions of any form suitable to the purpose, for example, powders suitable for pressing into tablets and granulates suitable for use in producing beverages. Examples of such compositions are given below. Exemplary tablets comprise fast-melt, chewable, and ingestible formulations. A formulation comprising a granulated "dry syrup" is also given below as an example of a formulation comprising a particulate of the present development from which a beverage can be prepared. The term "dry syrup" is used to denote a composition in powdery form that is dissolved in water to produce a potable liquid that includes components having desirable organoleptic activity.

### Examples

There are presented below examples of the preparation of compositions in accordance with the present development, including powdery forms thereof, and examples of comparative compositions. The examples include an evaluation of the following properties of particulates which are the subject of the examples: flowability; homogeneity; and density.

Flowability was evaluated by measurement of the angle of repose using a dynamic method. In flowability determinations, about 30 g of sample of particulate was dispensed from a 0.6 cm diameter tube having a funnel-shaped top with the bottom positioned 2 cm above a flat receiver. The angle of repose was calculated from the diameter of the circle covered by the dispensed sample. The procedure was carried out as detailed in Lieberman, H.A. and Lachman, L, "Pharmaceutical Dosage Forms: Tablets" Vol. 2, Marcel Dekker, NY, (1981) pp 26-29. It is preferred that particulates that are powders for use in preparing tablets exhibit an angle of repose, as determined by these measurements, of between about 28 degrees and about 45 degrees.

The homogeneity of the particulates was evaluated for appearance (visual homogeneity) and for consistency of the amount of bioactive material contained in randomly drawn aliquots (doses) of the particulates (dosage homogeneity).

Visual homogeneity was evaluated for those particulates in which the lipophilic liquid imparts a distinctive coloration to the particulates. A sample of particulate was considered to have visual homogeneity, when, viewed on a grain to grain comparison, the particulates had a uniform color (no significant lighter or darker grains). In samples where some grains of the particulate were markedly darker or lighter than the average tone of the sample, the particulate was considered to be visually non-homogeneous. Particulates of the examples were also evaluated visually to determine if they had an oily appearance. None of the exemplary particulates of the present development had any visual trace of oil on their surfaces.

Dosage homogeneity was determined by extracting the bioactive material from random samples of particulate into a solvent and quantifying the amount of bioactive material extracted via high pressure liquid chromatographic measurement using art recognized methodology. This was performed according to Uniformity of Dosage Unit, Monograph 905, beginning of page 2001 as described in the US Pharmacopoeia 24 (USP 24). The particulate was considered to have dosage homogeneity if 9 out of 10 randomly selected dosage units assay in the range of 85 % to 115 % of the calculated bioactive material content, or 10 out of 10 randomly selected dosage units assay in the range of 75 % to 125 % of the calculated bioactive material content of the average dose, for a set of measurements where the individual values display a correlation coefficient that indicates no greater than 6% variability between individual measurements. A composition comprising particulate material of the present invention is considered to have a liquid component dispersed uniformly throughout the composition if it demonstrates according to the above described test that it has dosage homogeneity.

In addition, the density of exemplary particulates was evaluated by obtaining bulk and tapped density measurement on a sample of particulate according to the procedure described in section 2.9.15 of the European Pharmacopoeia, 2001 edition (EP), and calculating from those measurements the Carr index of the particulate according to the procedure described in "Granulation, Tabletting, and Capsule Technology", pp 18-21, June 2001, The Hague Center for Professional Advancement. Bulk and tapped density measurements were carried out on an Erweka tapped volumeter apparatus in accordance with the EP and USP 24 listed procedures. A Carr index of less than 15 % was considered indicative of a preferred particulate for use in tablet formulations.

Various of the examples below also describe the preparation of tablets from a powdery form of particulates of the present development. The taste and following physical properties of the tablets were also evaluated: hardness; friability; homogeneity; and dispersibility in the case of fast-melt and ingestible composition.

Hardness was evaluated on a Schloninger tablet hardness testing apparatus according to the procedure described in Remington's Pharmaceutical Sciences (17^{th} Edition), Mack Publishing Co., 1985, pp. 1608-1609. For fast-melting composition tablets, a hardness ranging from about 1 (equal to 9,8 N) to about 10 kiloponds (Kp) was considered acceptable. For chewable tablets, a hardness range of about 1 to about 25 Kp was considered acceptable. And for ingestible tablets, a hardness range of about 10 Kp to about 20 Kp was considered acceptable.

Friability was determined using a Sotax F2 instrument according to the procedure described in the USP Pharmacopoeia, monograph 1216, beginning pg, 2148. The European Pharmacopoeia, 3^{rd} edition, lists acceptable values of friability for uncoated tablets as less than one wt. %.

Both visual and dosage homogeneity of the tablets were evaluated. Visual homogeneity was evaluated by inspecting samples of the tablets. Tablets that were free of spots remarkably darker than the average color of the tablet or free of a "stained" appearance were classified as being visually homogeneous. The presence of the aforementioned types of spots and staining are caused by the lipophilic liquid exuding into the various tablet excipients surrounding a grain of the powder. Dosage homogeneity of tablets were evaluated by determining the variability (if any) in the amount of bioactive material contained in the tablet according to the same procedure described above for determining dosage homogeneity in the particulates.

The dispersibility of fast-melting and ingestible tablets was evaluated using a Sotax DTX3 Disintegration tester according to the procedure described in Remington's Pharmaceutical Sciences (17^{th} Edition), Mack Publishing Company, 1985, pp. 1609-1610. Acceptable dispersion times for fast-melt tablets are generally less than about 15 minutes after immersion of the sample in the dispersing solvent. For ingestible tablets, acceptable dispersion times are generally less than about 30 minutes after immersion in the dispersing solvent.

Organoleptic properties (taste sensations) of the tablets were evaluated subjectively by a panel of testers. Samples of the tablets were administered to volunteers who rated them on an arbitrary scale of 1 to 5 for flavor and lack of objectionable taste sensations such as bitterness or anesthetic taste. The ratings in each category were averaged for all the testers. A formulation was determined to have acceptable taste when the average rating indicated that it had low or no objectionable taste sensations associated with it and, in addition, possessed a discernable degree of desirable taste sensations.

The first two groups of examples illustrate the preparation according to the present development of free-flowing particulates that comprise a lipophilic liquid.

The first group of two examples is illustrative of compositions prepared by placing the lipophilic liquid into an open bowl, adding thereto a hydrophilic solid dispersant, and mixing the ingredients manually by means of a spatula until a homogeneous mixture of the ingredients was obtained. The mixture had the form of a paste. A particulating agent was added then to the mixture and manual blending was continued until a dry, free-flowing particulate was obtained. Blending was carried out under ambient conditions, typically, a temperature of less than about 30 °C and about 35 % relative humidity.

The lipophilic liquid of the compositions of the first group of examples were oils derived from natural sources; they were all obtained from either Pharmabase or Geobell, AG, and used as received. The particulating agent was Aerosil A 200^{®} silica from Degussa. The hydrophilic solid dispersants used in the compositions of the first group of examples are identified below in Table 1. They were obtained from Roquette.

The constituents (and amounts) comprising the compositions of the first group of examples are identified below in Table 1.

**Table 1**

| Ex. No. | Lipophilic Liquid, 50 g | Hydrophilic Solid Dispersant, 50 g | Particulating Agent, wt. |
|---|---|---|---|
| 1 | lycopene oil | Mannitol | silica, 25 g |
| 2 | fennel oil | Mannitol | silica, 20 g |

The particulates comprising the constituents of Table 1 above were evaluated according to the procedures described above and determined to be non-oily, homogeneous, and free-flowing. They were further determined to have a Carr index of less than 15 % which is a range suitable for preparing tablets from the particulates.

The second group of examples describes compositions which were prepared by first placing the hydrophilic solid dispersant into an open bowl and adding gradually a lipophilic liquid with blending until all of the liquid had been added and a homogeneous mixture had been obtained. As with the first group of examples, the mixture was in the form of paste. Thereafter, a particulating agent was added. As with the first two examples, blending was carried out manually, using a spatula, under ambient conditions. The oils used in preparing the second group of examples were derived from natural sources and obtained as items of commerce from Geobell, AG. The oils were used as received. Mannitol was employed as the dispersant and silica was employed as the particulating agent; they were obtained from the same sources and the powders were prepared in the same manner as described above.

The constituents (and amounts) comprising the compositions of the second group of examples are identified below in Table 2.

**Table 2**

| Ex. No. | Lipophilic Liquid, 50 g | Hydrophilic Solid Dispersant, 50 g | Particulating Agent, Wt. |
|---|---|---|---|
| 3 | vitamin A oil | Mannitol | Silica, 25 g |
| 4 | juniper oil | Mannitol | Silica, 25 g |
| 5 | valerian oil | Mannitol | Silica, 20 g |

The compositions of Table 2 were also evaluated by the procedures described above. They were determined to be non-oily, free-flowing, homogeneous particulates. Based on density measurements as described above, they were also determined to be good candidates for incorporation into tablet formulations.

Taken together, the first and second groups of examples demonstrate that the lipophilic liquid and the hydrophilic solid dispersing agent can be added in any order in preparing a homogeneous mixture of the two, prior to blending with a particulating agent to form a composition of the present development.

The next example is comparative in nature.

### Example No. C-1

For comparative purposes, there was prepared a composition consisting of only a lipophilic liquid and a particulating agent. It was prepared in the manner described above for the previous examples. Thus 50 g of lycopene oil and 25 g of silica were blended in a bowl using a spatula. The resulting material produced a visually non-homogeneous, clumpy (non-free flowing) particulate solid. Addition of a hydrophilic solid dispersant (mannitol, 50 g) to this material did not improve the appearance or pour characteristics of the composition.

The next three groups of examples are illustrative of the formation of tablets containing powdery fractions of particulates (hereinafter, "powders") selected from those prepared in Example Nos. 1 to 5 and C-1 above. The first group of these examples illustrates the use of the powders prepared according to the present development in fast-melting tablets (intended to be dissolved in the buccal cavity) and, for comparison, a fast-melting tablet prepared from the powder of Example No. C-1 (lycopene oil absorbed on silica alone). The second group exemplifies the use of the powders in forming chewable tablets and the third group exemplifies the use of the powders in tablets intended for oral ingestion and delivery of bioactive ingredients to the gastrointestinal tract (hereinafter "ingestible tablets").

The examples in this group (Example Nos. 6 to 7) show the incorporation into rapidly dissolving tablet formulations of lycopene powder, juniper powder, and clove powder produced by the method of the present development and, for comparison, powder produced by the absorption of lycopene oil onto silica alone. Lycopene is administered commonly as an antioxidant. Juniper oil is administered as a diuretic agent and clove oil is administered as an analgesic. The examples demonstrate that these liquids can be incorporated into rapidly dissolving tablets after converting them to powders using the method of the present development. The tablets of these examples undergo rapid dissolution when placed into the buccal cavity. Rapid dissolution properties are imparted to the tablets by including in their formulation a composition (hereinafter "base powder") produced by granulating mannitol, sorbitol, and citric acid in an Aeromatic Strea 1 fluid bed granulator using a water/ polyethylene glycol (PEG 6000) granulating solution.

To prepare the base powder, measured amounts of mannitol, sorbitol, and citric acid were placed together into the cone of the fluid bed granulator and fluidized with air at a temperature of about 40 °C. About 30 ml of a granulating solvent comprising demineralized water and PEG 6000 were passed into the granulator at a rate of about 10 ml/minute. After all of the granulating solvent had been introduced into the granulator, the resulting granulated material was dried by continuing the flow of dry air for about 20 minutes at about 50 °C.

To prepare a composition for tableting, the base powder and the other dry constituents were placed into an Erweka cube blender and blended for 15 minutes. At the end of 15 minutes, an admixture of the constituents in the form of a homogeneous powder was obtained. Aliquots of this powder were then tableted in a Ronchi CT 20 eccentric tableting press fitted with a toroidal punch of 13 mm in diameter. The stroke of the press was adjusted to provide sufficient pressure to produce tablets displaying little friability under ordinary handling conditions.

### Example No. 6 - Tablets Containing Lycopene Powder

A fast-melting tablet base powder was prepared by granulating 30.38 g of mannitol, 19.71 g of sorbitol, and 2.16 g of citric acid together with a water solution containing 20 g of demineralized water and 1.08 g of PEG 6000 using the granulating procedure described above. The powder from which the tablets were formed was prepared in a blender by blending: (A) 53.33 g (dry weight) of the aforementioned fast-melting tablet base powder; (B) 41.67 g of lycopene powder prepared according to Example No. 1; and (C) 1.00 g of magnesium stearate, 2.00 g of wild berry flavor (Sensient) and 2.00 g of aspartame (HSC). The resulting homogeneous powder was removed from the blender and compressed into tablets weighing 500 mg using the equipment and procedure described above. Each tablet contained 15.00 mg of the lycopene oil used in the preparation of the lycopene powder.

The physical and organoleptic characteristics of the tablets were evaluated according to the procedures described above. The tablets had acceptable dissolution and taste characteristics. Additionally, they were found to have hardness and friability characteristics sufficient to endure the rigors of ordinary handling.

### Example No. 7 - Tablets Containing Juniper Powder

A fast-melting tablet base powder was prepared by granulating 40.10 g of mannitol, 26.02 g of sorbitol, and 2.85 g of citric acid together with a water solution containing 50 g of demineralized water and 1.43 g of PEG 6000 using the granulating procedure described above. The powder from which tablets were formed was prepared in a blender by blending: (A) 70.40 g (dry weight) of the aforementioned base powder; (B) 25.00 g of juniper powder prepared according to Example 4; and (C) 1.00 g of magnesium stearate, 1.60 g of fresh mint flavor (Givaudan) and 2.00 g of aspartame (HSC). The homogeneous powder was removed from the blender and compressed into tablets weighing 500 mg using the equipment and procedure described above. Each tablet contained 50.00 mg of juniper oil used in the preparation of the juniper powder of Example No. 4.

The physical and organoleptic characteristics of the tablets were evaluated according to the procedures described above. The tablets had acceptable dissolution and taste characteristics. Additionally, they were found to have hardness and friability characteristics sufficient to endure the rigors of ordinary handling.

The next example is comparative in nature.

### Example No. C-2

For comparison purposes, a fast-melting tablet (without a hydrophilic solid dispersant) was prepared by blending: (A) 53.33 g (dry weight) of the fast-melting tablet base powder prepared according to Example No. 6; (B) 41.67 g of lycopene oil absorbed onto silica, prepared according to Example No. C-1; and (C) 1.0 g of magnesium stearate, 2.0 g of wild berry flavor (Sensient) and 2.0 g of aspartame (HSC). Tablets were produced from this admixture using the same procedure and conditions used to prepare the tablets of Example No. 6.

The physical properties of these tablets were compared with those of the tablets of Example No. 6. Tablets of the present example fractured at a lower force than those of Example No. 6, the tablets of the present example fracturing at 1 Kp or less. They also displayed extreme friability in that the tablets of the present example shattered to the extent that their friability was not measurable. Visual comparison between the tablets of Example No. 6 and Example C-2 showed that lycopene oil exuded from the silica in the tablets of the present example, whereas no exudation was observed in the Example No. 6 tablets. Exuded lycopene oil in the tablets of the present example was particularly noticeable in those areas of the tablets that were subjected to the highest pressures during tableting, that is, at the junction between the rounded edge and the center web of the toroidal-shaped tablet, where streaks of lycopene oil were observed. Throughout all areas of tablets of the present example, spots of lycopene oil stained the tablets.

The next group of examples illustrates the incorporation of lecithin powder, vitamin A powder, and fennel powder prepared according to the method of the present development into ingestible tablets. Vitamin A oil is a dietary supplement. Lecithin and fennel oil can be used as diuretics or dietetic supplements. The examples demonstrate that these oils can be incorporated into ingestible tablets by first converting them into a particulate using the method of the present development.

In these examples (Nos. 8 to 9), a precursor of the granulated base composition of the tablet (granulate precursor) is prepared by drying at 85°C to 1 % relative humidity a solution comprising 20 g of Mays starch (Centonze), 80 g of demineralized water, and in amounts identified below, lactose and poly(vinyl pyrrolidone). The resulting solid is then granulated in a Aeromatic Strea 1 fluid bed granulator. The granulated material is sieved to isolate granulate which has a nominally 1.5 mm average diameter and which serves as the tablet base (hereinafter "granulated base composition").

The granulated base composition was converted to a powder from which tablets were made by blending it and other dry constituents, as described below, in an Erweka cube blender for 15 minutes. At the end of 15 minutes, a homogeneous powder was obtained. Aliquots of the powder were tableted using a Ronchi CT 20 eccentric tableting press fitted with a scored 8 mm diameter punch using sufficient pressure to produce tablets displaying little friability under ordinary handling conditions. The hardness, friability, and dissolution characteristics of the tablets of this group of examples were evaluated using the equipment and procedures described above.

### Example No. 8 - Tablets Containing Vitamin A Powder

A granulated base composition was prepared by adding to the granulate precursor described above for this group of examples 75.17 g of lactose and 2.5 g of poly(vinyl pyrrolidone). The resulting mixture was then dried and granulated.

Into an Erweka cube blender were placed 177.67 g of the granulated base composition, 1.40 g of vitamin A powder prepared according to Example No. 8, and 0.93 g of magnesium stearate. The dry materials were blended according to the procedure described above to produce a homogeneous powder admixture. The powder was tableted as described above into tablets weighing 200 mg. Each tablet contained 0.800 mg of the vitamin A used in preparing the vitamin A powder.

The resulting tablets were evaluated as described above and showed physical characteristics within acceptable ranges. The tablets were visually evaluated and found to have a regular surface and be devoid of edge erosion.

### Example No. 9 - Tablets Containing Fennel Powder

An ingestible tablet base composition was prepared by adding to the granulate precursor described above for this group of examples 74.17 g of lactose and 2.5 g of poly(vinyl pyrrolidone). The resulting mixture was then dried and granulated.

Into an Erweka cube blender were placed 100 g of the granulated base composition prepared for this example, 2.40 g of fennel powder prepared according to Example No. 3, and 0.93 g of magnesium stearate. The constituents were blended according to the procedure described above to yield a homogeneous powder admixture. The resultant powder was tableted as described above into tablets weighing 200 mg. Each tablet contained 2.00 mg of fennel oil used in preparing the fennel powder.

The resulting tablets were evaluated as described above and showed physical characteristics within acceptable ranges. The tablets were evaluated visually and found to have a regular surface and be devoid of edge erosion.

### Example No. 10 - Dispersible Granulate Containing Valerian Particulate

This example illustrates the use of a valerian oil particulate of the present development as a constituent in a granulate formulation ("dry syrup") which is dispersed into a hot liquid prior to consumption by the user. Valerian oil is exemplary of oils used in sedative formulations. This example demonstrates that such oils may be incorporated into particulates from which they may be dispersed readily if the oil is first incorporated into a solid form using the method of the present development.

A granulating solvent was prepared by combining about 30 g of demineralized water with about 1.00 g of Tween^{®} 80 (a polyglycolized ether having surface active properties, Fluka). A granular base composition was prepared by placing 88.57 g of sucrose (Zucker Muhle Rupperswill), 1.00 g of aspartame (HSC), 2.80 g of citric acid (Roche), 4.00 mg of Brilliant Blue (E133 Flachsmann), 8.00 mg of riboflavin 5-phosphate dehydrate (CFS), and 1.20 g of Mays starch (Centonze) into an Aeromatic Strea-1 fluid bed granulator. The dry constituents were fluidized using air at about 40 °C and granulated by introducing about 10 ml of the granulating solvent to the granulator over a period of about 2 minutes while maintaining a 40 °C temperature. After all of the granulation solvent had been introduced, the granulated material was dried by continuing the flow of fluidizing air for about 30 minutes at 50 °C.

Into an Erweka cube blender were placed 100 g of the dry granular base composition prepared for this example. To the dry granular base were added 5.00 g of valerian oil granulate prepared according to Example No. 10, 0.32 g of mint flavor (Givaudan), and 0.10 g of Aerosil A200^{®} (Degussa). The resulting mixture was blended for 15 minutes to obtain a homogeneous admixture. Aliquots of the admixture weighing 2.50 g were packaged into sachets. Each package contained 50.00 mg of the valerian oil used in preparing the valerian particulate.

The dissolution characteristics of the sachets were evaluated by placing them in demineralized water at ambient temperature (about 20 °C) and observing that the admixture dispersed completely within 60 seconds. The resulting liquid dispersion was evaluated for taste using the procedure described above. The results indicated that the sachets produced an acceptable tasting beverage.

## Claims

1. A method for forming a composition in solid particulate form from a mixture of constituents, at least one of which is a liquid, comprising forming said composition from a mixture of constituents comprising a lipophilic liquid, mannitol as hydrophilic solid dispersant, and silica as solid particulating agent wherein the lipophilic liquid and mannitol are combined in a weight ratio of 1:1 and wherein silica is combined with the mixture of lipophilic agent and mannitol in a weight ratio of silica to said mixture of 1:4.

2. A method of Claim 1 wherein said liquid includes a bioactive material.

3. A tablet produced by pressing, at a pressure which provides a cohesive tablet, an aliquot of a powder comprising an admixture of a fast-melting tablet base and a powder produced by the method according to Claim 1.

## Patentansprüche

1. Ein Verfahren zur Herstellung einer Zusammensetzung in fester partikulärer Form aus einer Mischung von Inhaltsstoffen, von denen wenigstens einer eine Flüssigkeit ist, umfassend das Bilden besagter Zusammensetzung aus einer Mischung von Inhaltsstoffen, umfassend eine lipophile Flüssigkeit, Mannitol als hydrophiles festes Dispergiermittel, und Siliciumdioxid als festes Partikulierungsmittel, wobei die lipophile Flüssigkeit und Mannitol in einem Gewichtsverhältnis von 1:1 kombiniert werden und worin Siliciumdioxid mit der Mischung aus lipophilem Agens und Mannitol in einem Gewichtsverhältnis des Siliciumdioxids zu der besagten Mischung von 1:4 kombiniert wird.

2. Ein Verfahren nach Anspruch 1, wobei die besagte Flüssigkeit ein bioaktives Material einschließt.

3. Eine Tablette, hergestellt durch Pressen eines Aliquots eines Pulvers, umfassend eine Beimischung eines schnellschmelzenden Tablettengrundstoffs und eines Pulvers, das nach dem Verfahren gemäß Anspruch 1 hergestellt wurde, bei einem Druck, der eine zusammenhaltende Tablette liefert.

## Revendications

1. Procédé pour former une composition sous forme solide particulaire à partir d'un mélange de constituants, dont au moins l'un est un liquide, comprenant la formation de ladite composition à partir d'un mélange de constituants comprenant un liquide lipophile, du mannitol en tant que dispersant solide hydrophile, et de la silice en tant qu'agent de formation de particule solide, dans lequel le liquide lipophile et le mannitol sont combinés en un rapport pondéral de 1:1 et dans lequel la silice est combinée avec le mélange d'agent lipophile et de mannitol en un rapport pondéral de la silice audit mélange de 1:4.

2. Procédé selon la revendication 1, dans lequel ledit liquide inclut une matière bioactive.

3. Comprimé fabriqué par pressage, à une pression qui fournit un comprimé cohésif, d'une fraction aliquote d'une poudre comprenant un mélange d'une base pour comprimé fondant rapidement et d'une poudre fabriquée par le procédé selon la revendication 1.
